# EUROPÄISCHE PATENTSCHRIFT

(11) **EP 0 805 797 B1**
(45) Veröffentlichungstag und Bekanntmachung des Hinweises auf die Patenterteilung: **07.04.1999**
(21) Anmeldenummer: 96900963.8
(22) Anmeldetag: 17.01.1996
(51) Int. Cl.: C07C 239/10, C07C 251/48, C07C 255/64, C07D 213/76, C07D 213/80, C07D 215/42, C07D 215/40, C07C 239/12, C07D 249/12, C07D 231/22, C07D 471/04

(54) **VERFAHREN ZUR HERSTELLUNG VON N-ARYL- UND N-HETARYLHYDROXYLAMINEN**
PROCESS FOR PRODUCING N-ARYLHYDROXYLAMINES AND N-HETARYLHYDROXYLAMINES
PROCEDE DE PRODUCTION DE N-ARYLHYDROXYLAMINES ET DE N-HETARYLHYDROXYLAMINES

(30) Priorität: 28.01.1995 DE 19502700
(43) Veröffentlichungstag der Anmeldung: 12.11.1997
(73) Patentinhaber: BASF AKTIENGESELLSCHAFT, 67056 Ludwigshafen (DE)
(72) Erfinder: GÖTZ, Norbert, D-67547 Worms (DE); MÜLLER, Bernd, D-67227 Frankenthal (DE); SAUTER, Hubert, D-68167 Mannheim (DE); GEBHARDT, Joachim, D-67157 Wachenheim (DE); WAGNER, Oliver, D-66450 Bexbach (DE)
(86) Internationale Anmeldenummer: EP9600170
(87) Internationale Veröffentlichungsnummer: WO9622967

(56) Entgegenhaltungen:
- EP-A- 0 147 879
- DE-A- 2 455 887
- GB-A- 1 092 027
- CHEM. IND. (DEKKER) (1988), 33(CATAL. ORG. REACT.), 135-47, XP000569987 KOSAK, JOHN R.: "Hydrogenation of nitroarenes - the hydroxylamine intermediate"

## Beschreibung

Die vorliegende Erfindung betrifft ein Verfahren zur Herstellung von aromatischen oder heteroaromatischen Hydroxylaminen der allgemeinen Formel I 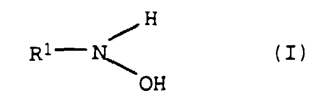 in der R¹ einen unsubstituierten oder substituierten Arylrest oder einen unsubstituierten oder substituierten Hetarylrest aus der Gruppe der Pyridine oder Chinoline darstellt, durch Hydrierung von Nitroverbindungen der allgemeinen Formel II

R¹-NO₂ (II)

in der R¹ die oben angegebene Bedeutung hat, in Gegenwart eines Platinkatalysators auf einem Aktivkohleträger oder in Gegenwart eines mit Schwefel oder Selen dotierten Palladiumkatalysators auf einem Aktivkohleträger, indem die Reaktion in Gegenwart einer am Stickstoffatom substituierten Morpholinverbindung der allgemeinen Formel III durchgeführt wird, 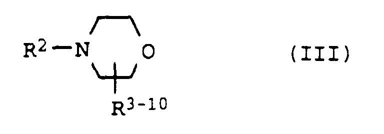 wobei R² Alkylreste mit 1 bis 5 Kohlenstoffatomen und R³ bis R¹⁰ Wasserstoffatome oder Alkylreste mit 1 bis 5 Kohlenstoffatomen bedeuten.

Die katalytische Hydrierung von aromatischen Nitroverbindungen zu N-Phenylhydroxylaminen ist seit langer Zeit bekannt (Houben-Weyl "Methoden der Org. Chemie" Bd. 10/1, S. 1155 - 1157; Bd. E 16a, Teil 1, S. 49 - 53). Im Vergleich zur relativ teueren elektrochemischen Reduktion und zur Reduktion mit Metallen, wie beispielsweise mit Zinkstaub, Amalgamen u. a., die eine ungünstige Abfallstoffbilanz aufweisen, ist die katalytische Hydrierung aus wirtschaftlicher Sicht die günstigste Methode. Ein Problem stellen bei diesem Reaktionstyp die Weiterreaktion zum aromatischen Amin, der stabilen Endstufe, und die Disproportionierung des gebildeten N-Phenylhydroxylamins zu den entsprechenden Nitrosoverbindungen und Anilinen dar. Aus diesen unerwünschten Zwischen- und Folgeprodukten können durch Folgeumsetzungen höhermolekulare Nebenprodukte, wie Azoxybenzole, Azobenzole und Hydrazobenzole gebildet werden, die beispielsweise durch Kondensation von Nitrosobenzolen und N-Phenylhydroxylaminen und Weiterreaktion der gebildeten Azoxybenzole entstehen können und unter Umständen erhebliche Ausbeuteverluste verursachen.

In der Regel werden Pd- oder Pt-Katalysatoren für diese Hydrierreaktionen empfohlen. Um Ausbeuten bzw. Selektivitäten > 50 % zu erreichen, sind nach derzeitigem Kenntnisstand Katalysatorzusätze in Form von Dimethylsulfoxid, divalenten Schwefelverbindungen oder verschiedenen organischen Phosphorverbindungen erforderlich (EP 85890, EP 86363, EP 147879, USP 3694509, EP 212375).

Mit diesen Zusätzen wird die Verbesserung der Selektivität durch Herabsetzen der Reaktionsgeschwindigkeit erreicht, was wiederum zu langen Reaktionszeiten führt. Weiterhin hat die teilweise Vergiftung bzw. Inaktivierung des Katalysators durch die Zusätze zur Folge, daß der Katalysator meistens schon nach einem Cyclus seine Aktivität verloren hat und erneuert werden muß.

In GB-A 1 092 027 wird die katalytische Hydrierung von Nitrocyclohexan zu N-Cyclohexylhydroxylamin in Gegenwart von primären oder sekundären Aminen wie z.B. Morpholin beschrieben. Cyclohexylhydroxylamin wird dabei in einer Ausbeute von 50 bis 70 % gewonnen.

Eine weitere Methode zur Herstellung von Phenylhydroxylaminen ist die katalytische Hydrierung von Nitroaromaten in Gegenwart von organischen Stickstoffbasen, wie Piperidin, Pyrrolidin, Pyridin u. a., die -bezogen auf das Einsatzprodukt- im Überschuß eingesetzt werden müssen (DE-OS 2 455 238, DE-OS 2 455 887, DE-OS 2 357 370, DE-OS 2 327 412). Die nach diesem Verfahren erreichbaren Ausbeuten liegen nach entsprechender Aufarbeitung und Reinigung bei 80 - 85 %. Nachteilig ist, daß sich mit dieser Variante nur relativ einfache Alkyl- und Chlornitrobenzole zu den entsprechenden Phenylhydroxylaminen hydrieren lassen. Abgesehen von einer Verbindung mit einem 1,3,4-Oxadiazolsubstituenten wird die Hydrierung komplizierterer Systeme nach dieser Methode nicht beschrieben.

Aufgabe der vorliegenden Erfindung war es, ein Verfahren zur Herstellung von aromatischen und heteroaromatischen Hydroxylaminen bereitzustellen, das einfach durchführbar ist, auf komplizierte Systeme anwendbar ist und eine hohe Selektivität und Ausbeute aufweist.

Demgemäß wurde das eingangs beschriebene Verfahren gefunden.

Es ist überraschend und war nicht vorhersehbar, daß die Partialhydrierung von aromatischen und heteroaromatischen Nitroverbindungen zu den entsprechenden Hydroxylaminderivaten nur in N-Alkylmorpholinen (N-subst. Tetrahydro-1,4-oxazinen) als Lösungsmittel zu optimalen Ausbeuten und Selektivitäten führt, während nach DE-OS 24 55 238 org. Stickstoffbasen, wie Pyrrolidine, Piperidine, Aniline oder Pyridine als beste Lösungsmittel für diese spezielle Hydrierreaktion empfohlen werden. Vergleichsversuche (s. Beispiele 1 d - i) zeigen eindeutig, daß die N-Alkylmorpholine bei dieser speziellen Hydrierreaktion anderen Lösungsmittelsystemen, wie Pyrrolidinen, Piperidinen, Pyridinen u. a. überlegen sind. In ihrer Abstufung liefern tertiäre Amine bessere Ergebnisse als sekundäre Amine. Am wenigsten geeignet sind in diesem Falle primäre Amine, da sie in der Regel Selektivitäten < 80 % zur Folge haben.

Da die als Reaktionsprodukte anfallenden Hydroxylaminderivate sehr labile Verbindungen sind, die sich nur schwer reinigen lassen und sich bei Einwirkung von Temperaturen > 100°C relativ schnell zersetzen, ist es umso bedeutender, daß sie nach dem erfindungsgemäßen Verfahren bei weitgehend quantitativem Umsatz in hoher Reinheit anfallen, so daß eine aufwendige Reinigung durch Abtrennung von Ausgangsprodukt und Nebenprodukten, wie den entsprechenden Anilinen, Azoxybenzolen u. a. entfällt.

Gegenüber dem Stand der Technik hebt sich das erfindungsgemäße Verfahren weiterhin dadurch ab, daß es sehr breit anwendbar ist. So lassen sich beispielsweise unterschiedlich substituierte heteroaromatische Nitroverbindungen genauso problemlos selektiv hydrieren, wie komplizierte Nitroaromaten mit Benzylether- oder Oximetherstrukturen, die in der Regel bei katalytischen Hydrierungen leicht unerwünschte Veränderungen erleiden (s. Beispiel 3).

Die als Ausgangsprodukte benötigten Nitroaromaten bzw. -heteroaromaten sind gut zugänglich und ihre Herstellung ist vielfach und ausführlich beschrieben (s. Houben-Weyl, Bd. 10/1, S. 463 - 889 und Bd. E 16 d/Teil 1, S. 255 - 405).

Für das erfindungsgemaße Verfahren eignen sich sowohl einfache Nitroverbindungen, wie beispielsweise 2-Methylnitrobenzol, 2-Benzylnitrobenzol, 2,3-Dichlornitrobenzol, 2-Methyl-3-fluornitrobenzol, 2-Methyl-1-nitronaphthalin, 2-Chlor-3-nitropyridin, 2-Methyl-8-nitrochinolin, als auch komplexere Verbindungen, die noch zusätzlich hydrierempfindliche Strukturen, wie Benzylether-, Oximether- sowie Ketogruppen oder heteroaromatische Substituenten besitzen.

Die nach dem Verfahren der Erfindung eingesetzten Katalysatoren enthalten Platin oder Palladium auf einem Kohleträger. Bei Verwendung eines Palladiumkatalysators muß dieser mit Schwefel oder Selen dotiert sein, um ausreichende Selektivität zu erhalten (siehe Beispiel 1c). Platin liefert beim Einsatz nach dem erfindungsgemäßen Verfahren ohne zusätzliche Dotierung ausgezeichnete Resultate. Eine Dotierung, beispielsweise mit Schwefel, hat keine nennenswerte Selektivitätsänderung mehr zur Folge. Die Katalysatoren können nach einem Reaktionscyclus abfiltriert und ohne spürbaren Aktivitätsverlust beim Folgeansatz wieder verwendet werden. Bei anderen Verfahren, die mit Zusätzen wie Dimethylsulfoxid, Dimethylaminopyridin oder org. Phosphorverbindungen arbeiten (EP 86363, EP 85890, EP 147879), gibt es in der Regel einen schnellen Aktivitätsabfall durch Vergiftung der Katalysatoren. Der Platin- bzw. Palladiumgehalt des Katalysators ist nicht kritisch und kann in weiten Grenzen variiert werden.

Zweckmäßig ist ein Gehalt von 0,1 bis 15 Gew.-%, vorzugsweise von 0,5 bis 10 Gew.-%, bezogen auf das Trägermaterial Kohle. Die Menge des eingesetzten Platins oder Palladiums beträgt zwischen 0,001 und 1 Gew.-%, bevorzugt zwischen 0,01 und 0,1 Gew.-%, bezogen auf die Nitroverbindung. In der bevorzugten Ausführungsform einer diskontinuierlichen Hydrierung wird der Katalysator als Pulver eingesetzt. Andere Träger wie Al₂O₃, SiO₂, BaSO₄, führen zu wesentlich schlechteren Resultaten (s. Beispiel 1k). Von entscheidender Bedeutung für das Erzielen von sehr guten Ausbeuten ist die Wahl der richtigen Lösungsmittel, da diese die Aktivität der Katalysatoren dergestalt beeinflussen, daß eine hohe Selektivität bei der Hydrierung von Nitroverbindungen zu den Hydroxylaminderivaten erreicht wird. Erfindungsgemäß sind diese Lösungsmittel ausschließlich tert. Amine mit Morpholinstruktur, wie beispielsweise 4-Methylmorpholin, 4-Ethylmorpholin, 4-Propylmorpholin, 4-Butylmorpholin, 4-Isobutylmorpholin, 4-Tertiärbutylmorpholin, 4-Pentylmorpholin, 4-Isopentylmorpholin, 2,4,6-Trimethylmorpholin, 2,3,4,5,6-Pentamethylmorpholin, 2,2,4,6,6-Pentamethylmorpholin.

In der Regel wird das tert. Amin im Überschuß eingesetzt, d. h. das Gewichtsverhältnis von tert. Amin zur Nitroverbindung ist größer als 1.

Der gewählte Temperaturbereich für die Partialhydrierung liegt zwischen - 20°C und + 100°C, vorzugsweise zwischen - 5 und + 50°C. Um Überhydrierungen zu vermeiden, wird bei der Temperatur, bei der die Hydrierung ausreichend schnell abläuft, ein Druck eingestellt, der zwischen Normaldruck und 10 bar Überdruck liegt. Normalerweise wird der Wasserstoff bei Normaldruck bzw. leicht erhöhtem Druck in den Hydrierreaktor eingegast. Weitere Lösungs- bzw. Verdünnungsmittel, wie beispielsweise Alkohole oder Ether, dürfen während der Hydrierung nicht anwesend sein, da sie ein drastisches Absinken der Ausbeute zur Folge haben (s. Beispiel 1j).

Die nach dem Verfahren der Erfindung hergestellten Verbindungen lassen sich als Aryl- bzw. Hetarylhydroxylamine mit Hilfe der Bamberger-Umlagerung in wertvolle substituierte Aromaten bzw. Heteroaromaten überführen (Houben-Weyl, Methoden der Organischen Chemie, Bd. 10/1, S. 1249 - 1251). Weiterhin stellen sie wichtige Zwischenpunkte für die Herstellung von Wirkstoffen in Pflanzenschutzmitteln dar (WO 93/15046).

Zum Teil wurden nach dem erfindungsgemäßen Verfahren auch neue Stoffe hergestellt, die nach anderen Methoden bisher nicht gewonnen werden konnten.

### Beispiel 1

### Herstellung von N-(o-Tolyl)-hydroxylamin

a) In einen 250-ml-Hydrierkolben mit Begasungsrührer gab man eine Lösung von 13,7 g o-Nitrotoluol in 100 ml N-Methylmorpholin und 0,36 g eines Katalysators, der 5 Gew.-% Platin auf Kohle (Typ F 103 RS/W von Degussa) enthielt. In diese Mischung wurde unter starker Rührung bei 28 bis 30°C solange Wasserstoff eingeleitet, bis keine Wasserstoffaufnahme mehr erfolgte (nach ca. 2 Stunden und 4,5 1 Wasserstoffverbrauch).
   Nach HPLC-Analyse des Rohproduktes war die Umsetzung zu 96,4 % zum N-(o-Tolyl-)hydroxylamin abgelaufen, die restlichen 3,6 % bestanden überwiegend aus nicht umgesetzten o-Nitrotoluol.
   Nach Abfiltration des Katalysators, Entfernen des N-Methylmorpholins im Vakuum und Kristallisation des Rückstandes aus Petrolether wurden 11,3 g N-(o-Tolyl-)hydroxylamin (Fp. 38 - 39° C) erhalten.
   Ausbeute:
   92 % d. Th.
   NMR (DMSO-d6, δ in ppm):
   8,25 (s, 1H), 7,9 (s, 1H), 7,1 (m, 2H), 6,95 (d, 1H), 6,7 (m, 1H)
b) Es wurde völlig analog a) gearbeitet, aber N-Methylmorpholin durch N-Ethylmorpholin ersetzt. Das isolierte Rohprodukt enthielt nach HPLC-Analyse 95,9 % N-(o-Tolyl-)hydroxylamin.
c) Es wurde analog a) verfahren, aber ein Katalysator der Zusammensetzung 5 Gew.-% Palladium, 0,2 Gew.-% Selen auf Kohle (Typ HO-51 der BASF) eingesetzt. Das isolierte Rohprodukt enthält nach HPLC-Analyse 94,2 % N-(o-Tolyl-)hydroxylamin.
d) Es wurde analog a) verfahren, aber N-Methylmorpholin durch N-Methylpiperidin ersetzt. Das isolierte Rohprodukt enthält nach HPLC-Analyse 86,7 % N-(o-Tolyl-)hydroxylamin, der Rest war überwiegend o-Toluidin.
f) Es wurde analog a) gearbeitet, aber N-Methylmorpholin durch Pyridin ersetzt. Das isolierte Rohprodukt enthielt nach HPLC-Analyse 85,6 %N-(o-Tolyl-)hydroxylamin, der Rest bestand überwiegend aus o-Toluidin.
g) Es wurde analog a) gearbeitet, aber N-Methylmorpholin durch Piperidin ersetzt. Das isolierte Rohprodukt enthielt nach HPLC-Analyse 44,9 % N-(o-Tolyl-)hydroxylamin, 4,5 % o-Toluidin, der Rest war überwiegend die Azoxyverbindung: 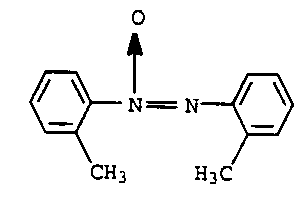
h) Es wurde analog a) gearbeitet, aber N-Methylmorpholin durch N,N-Dimethylisopropylamin ersetzt. Das isolierte Rohprodukt enthielt nach HPLC-Analyse 62,4 % N-(o-Tolyl-)hydroxylamin, 9,6 %o-Toluidin, der Rest war überwiegend die Azoxyverbindung 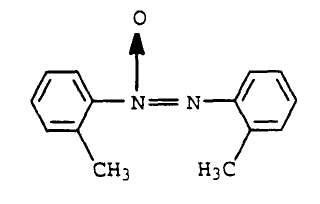
i) Es wurde analog a) gearbeitet, aber N-Methylmorpholin durch tert.-Butylamin ersetzt. Das isolierte Rohprodukt enthielt nach HPLC-Analyse 60,2 % N-(o-Tolyl-)hydroxylamin, 21,6 % o-Toluidin, der Rest war überwiegend die Azoxyverbindung 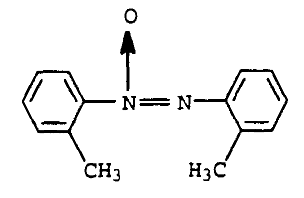
j) Es wurde analog a) gearbeitet, aber die Lösungsmittelmenge von 100 ml N-Methylmorpholin wurde durch eine Mischung aus 70 ml Methanol und 30 ml N-Methylmorpholin ersetzt. Das isolierte Rohprodukt enthielt nach HPLC-Analyse 58,6 % N-(o-Tolyl-)hydroxylamin, 26,3 %o-Toluidin, der Rest bestand noch überwiegend aus dem Ausgangsprodukt o-Nitrotoluol.
k) Es wurde analog a) verfahren, aber ein Katalysator der Zusammensetzung 5 % Platin auf Aluminiumoxid (von Engelhard) eingesetzt. Das isolierte Rohprodukt enthielt nach HPLC-Analyse 9,9 % N-(o-Tolyl-)hydroxylamin, 2,9 % o-Toluidin, der Rest bestand noch überwiegend aus dem Ausgangsprodukt o-Nitrotoluol.
l) Es wurde analog a) gearbeitet, aber ein Katalysator der Zusammensetzung 5 % Palladium auf Aktivkohle (HO-50 der BASF) eingesetzt. Das isolierte Rohprodukt enthielt nach HPLC-Analyse 52,5 % N-(o-Tolyl-)hydroxylamin, der Rest bestand über> wiegend aus o-Toluidin.
m) Es wurde analog a) gearbeitet, aber als Katalysator Raney-Nickel eingesetzt. Das isolierte Rohprodukt enthielt nach HPLC-Analyse 58,3 % N-(o-Tolyl-)hydroxylamin, 16,8 % o-Toluidin und 24,9 %o-Nitrotoluol.

### Beispiel 2

### Herstellung von N-(2,3-Dichlorphenyl)-hydroxylamin

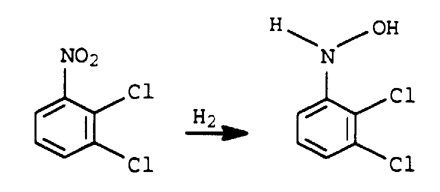

In einen 1,2-l-Hydrierautoklaven gab man eine Lösung von 134,4 g 2,3-Dichlornitrobenzol in 700 ml 4-Methylmorpholin und 5 g eines Katalysators, der 5 Gew.-% Platin auf Kohle (Typ F 103 RS/W von Degussa) enthielt. Nun wurde in diese Mischung unter intensiver Rührung bei 25 bis 32°C und 0,7 bar Wasserstoffdruck solange Wasserstoff eingeleitet, bis keine Wasserstoffaufnahme mehr erfolgte (nach ca. 9 Stunden und 32,4 1 Wasserstoffverbrauch).

Der Reaktionsaustrag wurde mit Tierkohle abfiltriert, das Filtrat im Vakuum (bei ca. 30 mbar) vom 4-Methylmorpholin befreit, wonach das Produkt zu kristallisieren begann. Nach zweimaliger Wäsche mit je 300 ml Cyclohexan und anschließender Trocknung wurden 116,3 g N-(2,3-Dichlorphenyl)-hydroxylamin erhalten.
Ausbeute:
93,3 % d. Th.
NMR (DMSO-d6, δ in ppm):
8,8 (s, 1H), 8,5 (s, 1H), 7,2 (m, 2H), 7,0 (dd, 1H)

### Beispiel 3

Umsetzung

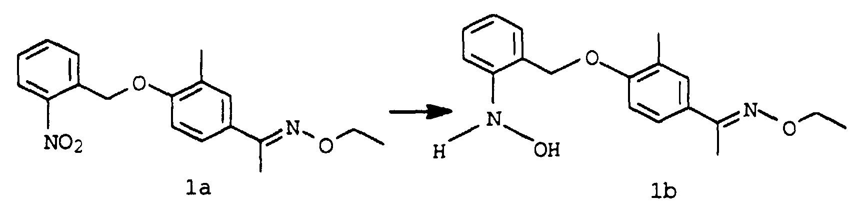

In eine 4-l-Hydrierapparatur gab man eine Lösung von 410 g la in 2,8 1 4-Methylmorpholin und 12 g Katalysator mit der Zusammensetzung 5 Gew.-% Platin auf Kohle (Typ F 103 RS/W von Degussa). In diese Mischung wurde nun unter intensiver Rührung bei 24 bis 29°C und 0,1 bar Wasserstoffdruck solange Wasserstoff eingeleitet, bis keine Wasserstoffaufnahme mehr erfolgte (nach ca. 4 h und 57,5 1 Wasserstoffverbrauch).

Der Reaktionsaustrag wurde mit Tierkohle abfiltriert, das Filtrat am Rotationsverdampfer (im Vakuum bei 25 - 30 mbar und 65°C Badtemperatur) eingeengt und der Rückstand durch Behandlung mit 1,2 1 Pentan zum Kristallisieren gebracht.

Die Kristalle wurden abfiltriert, mit etwas Pentan nachgewaschen und getrocknet. Man erhielt 359,2 g der Verbindung lb, entspr. einer Ausbeute von 91,5 % d. Th.
NMR (DMSO-d6, δ in ppm):
8,45 (s, 1H), 8,1 (s, 1H), 7,5 (s, 1H), 7,45 (d, 1H), 7,25 (m, 3H), 7,0 (d, 1H), 6,85 (m, 1H), 5,05 (s, 2H), 4,15 (q, 2H), 2,25 (s, 3H), 2,15 (s, 3H), 1,25 (t, 3H)

Analog den Beispielen la, 2 bzw. 3 wurden folgende Verbindungen hergestellt, die die Anwendungsbreite des erfindungsgemäßen Verfahrens dokumentieren.

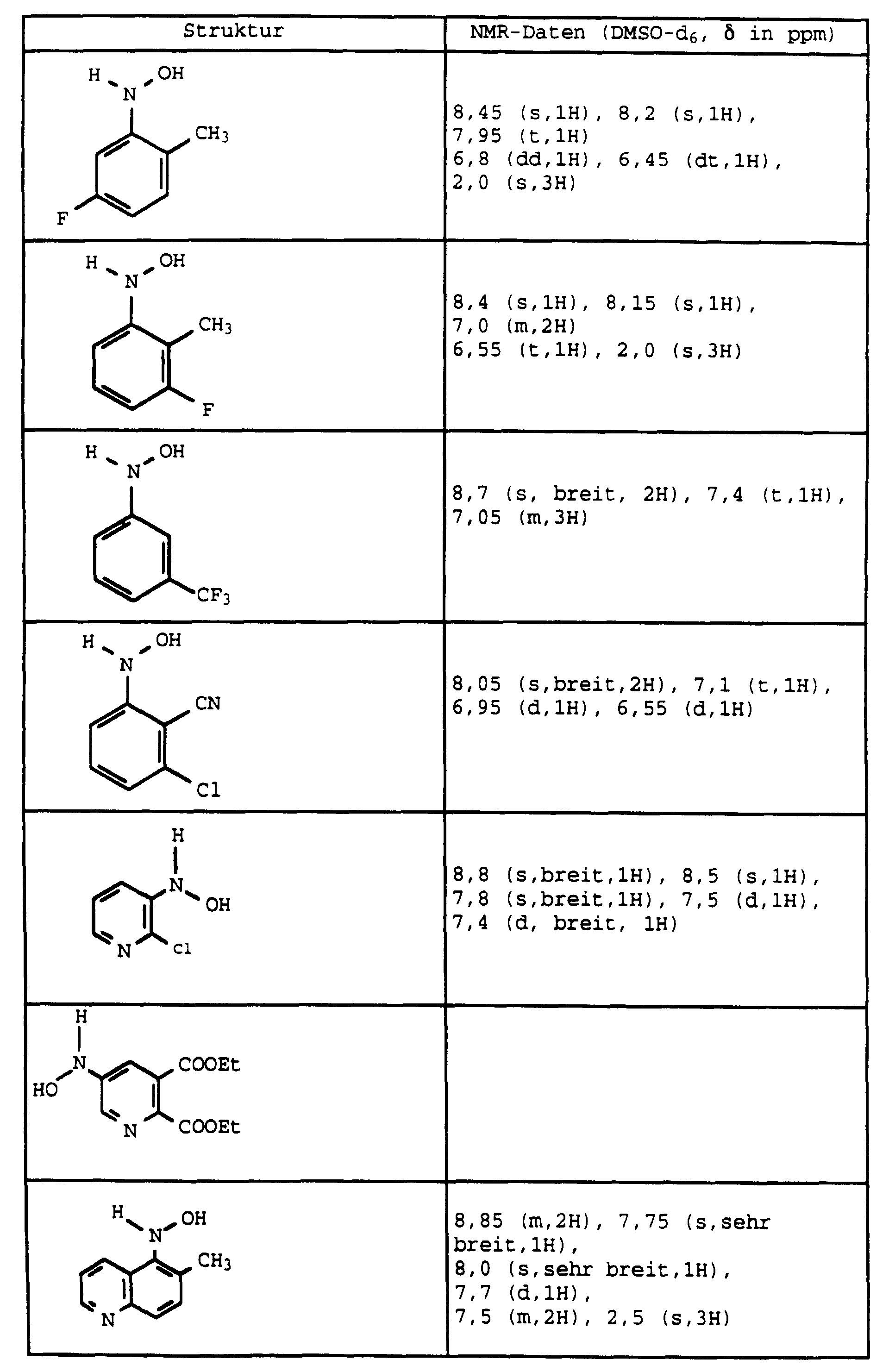

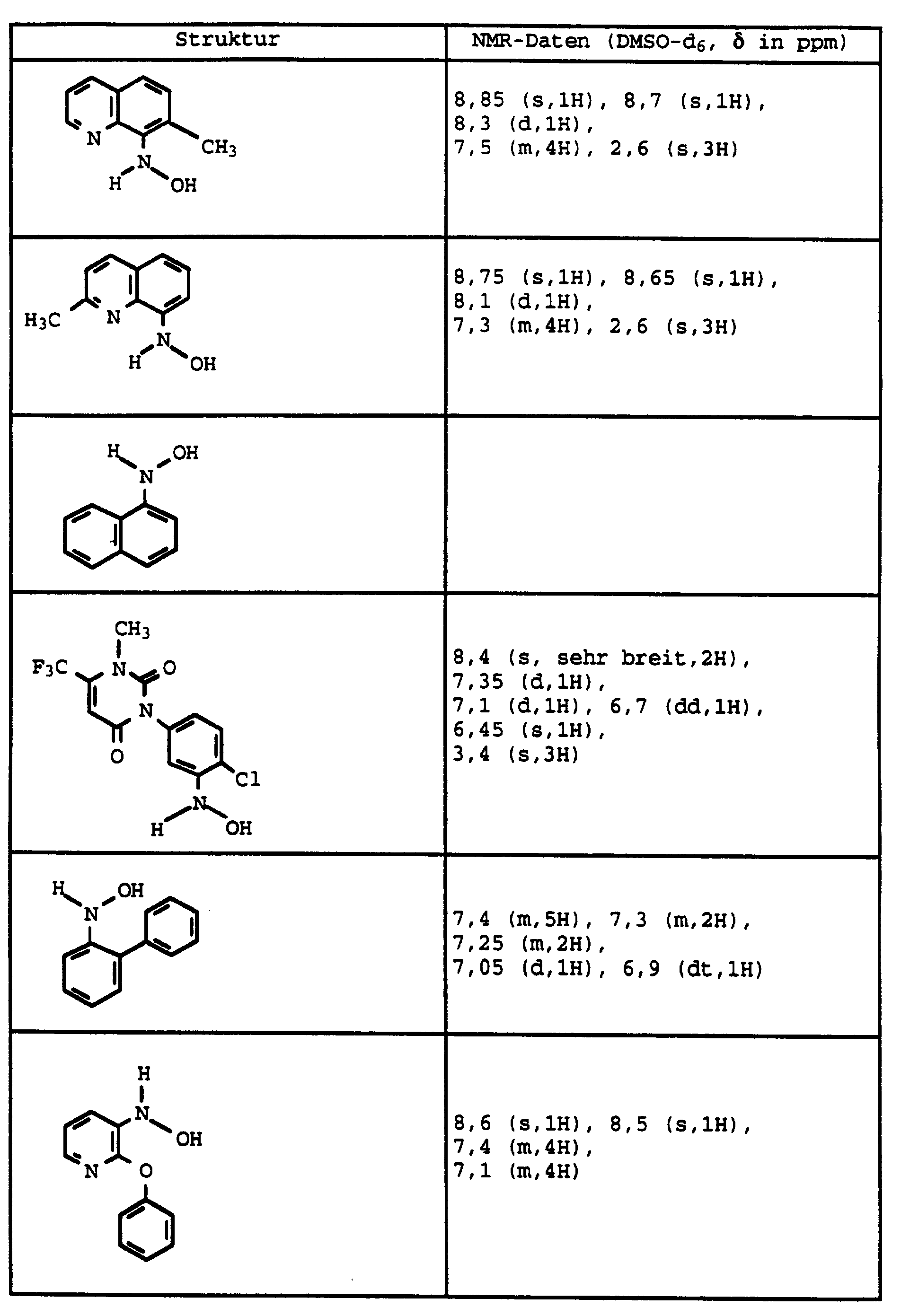

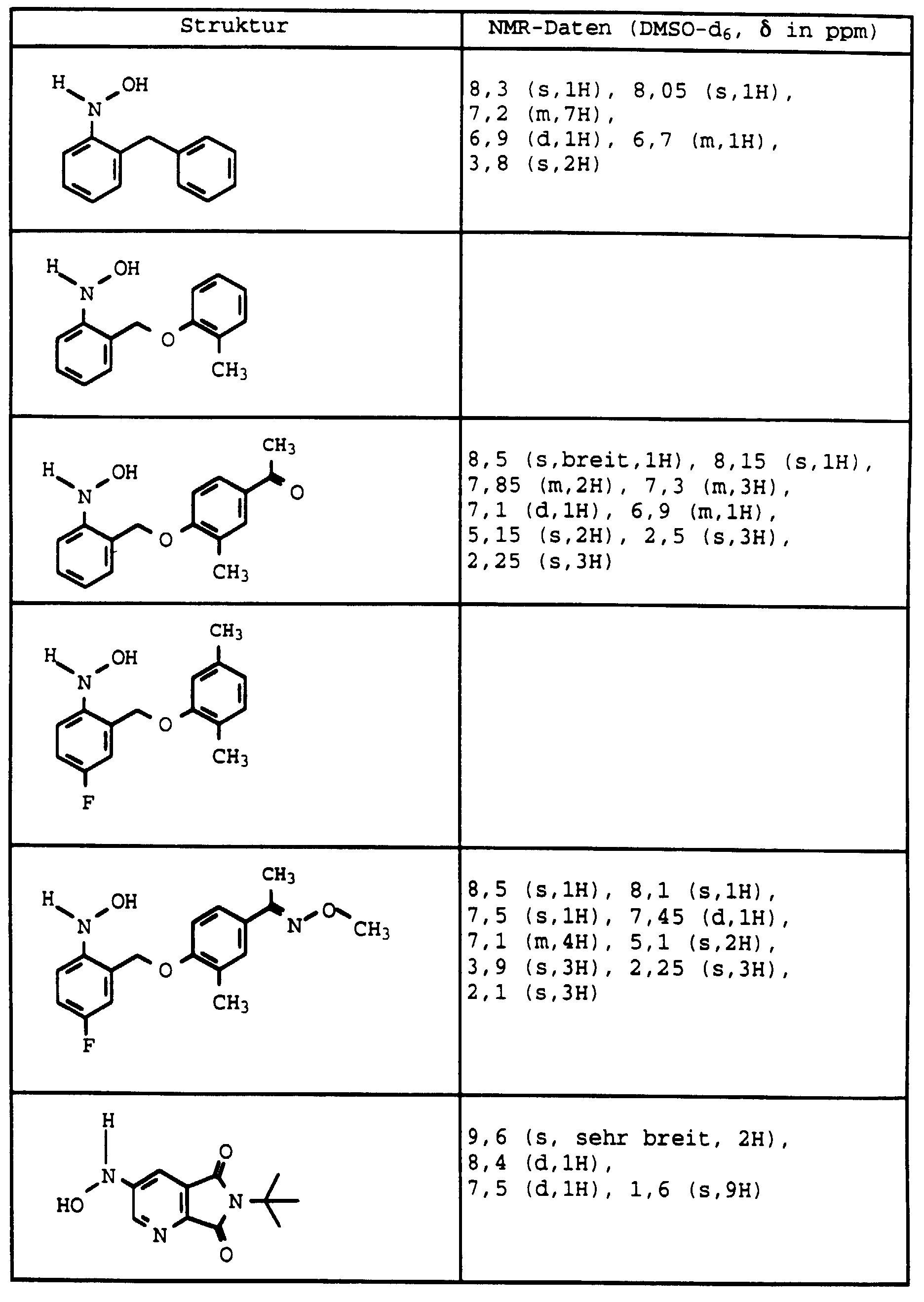

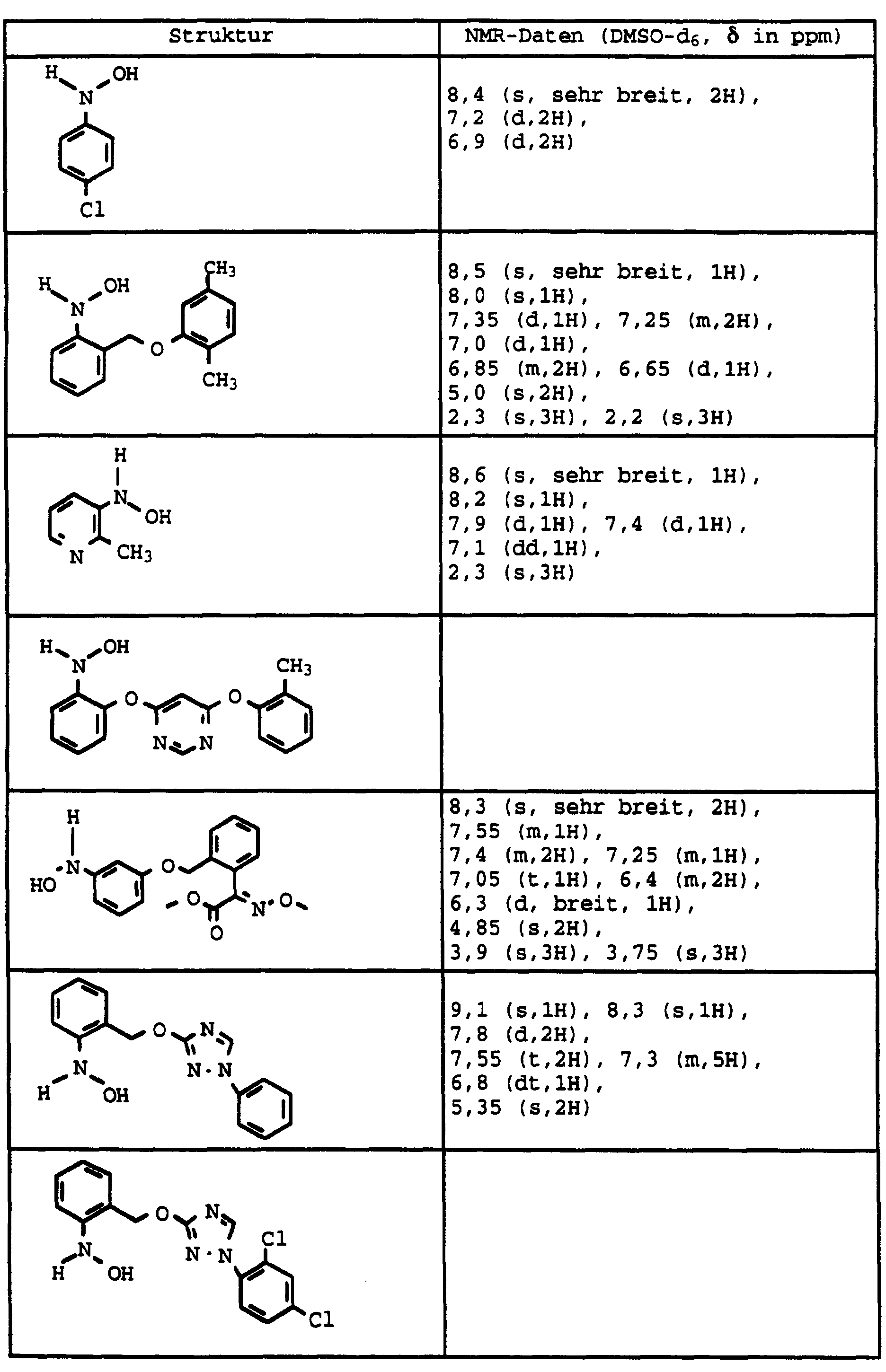

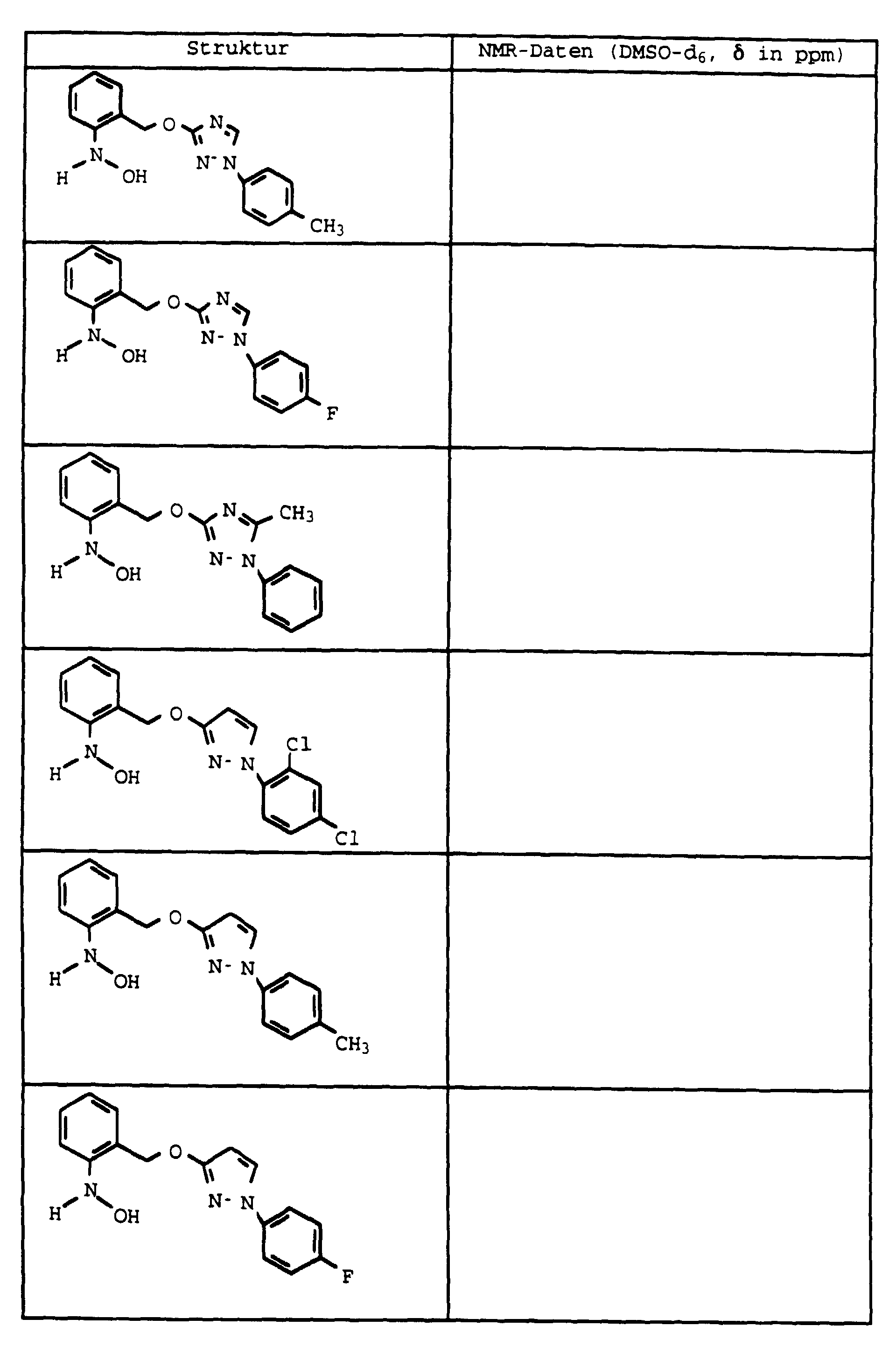

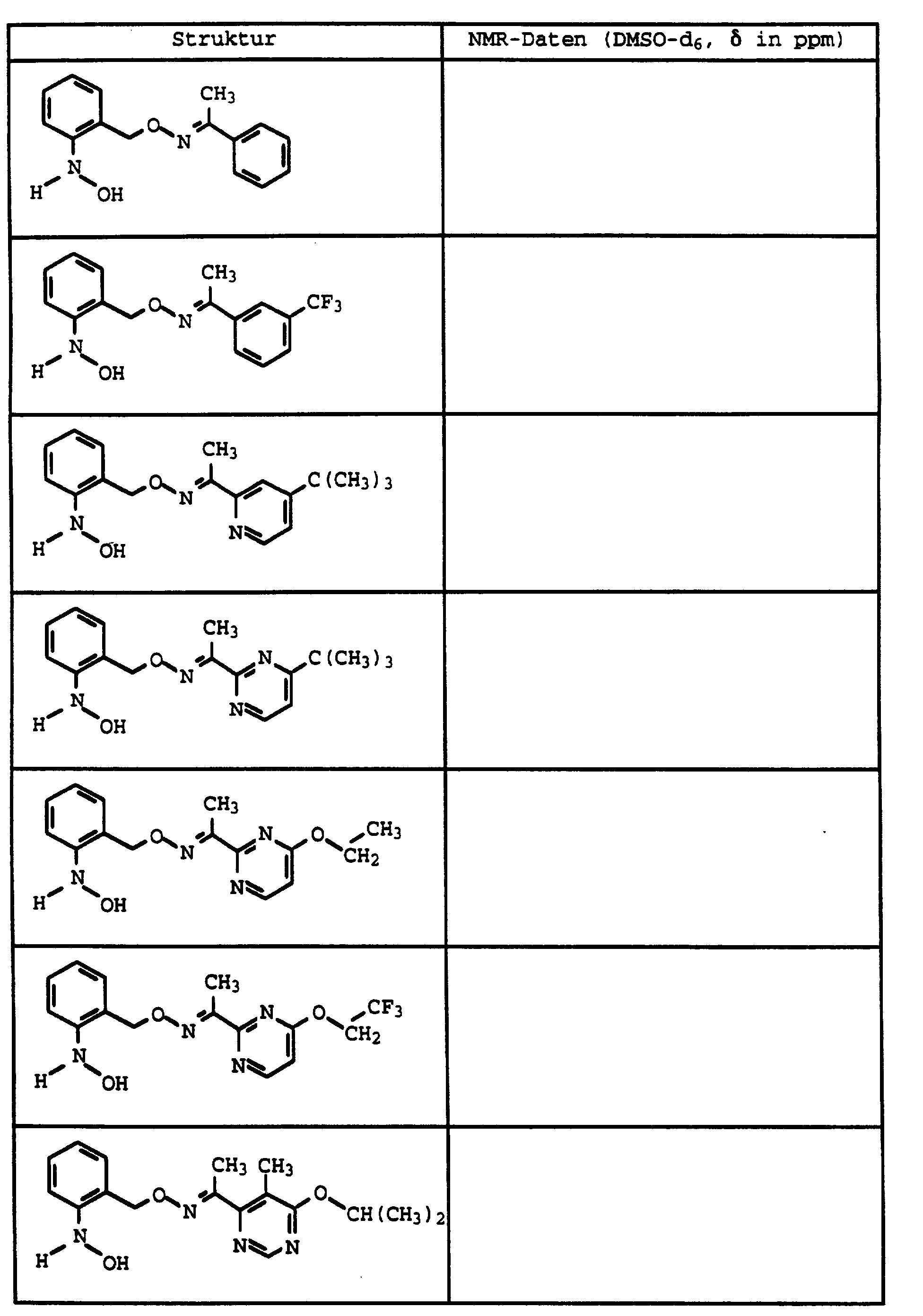

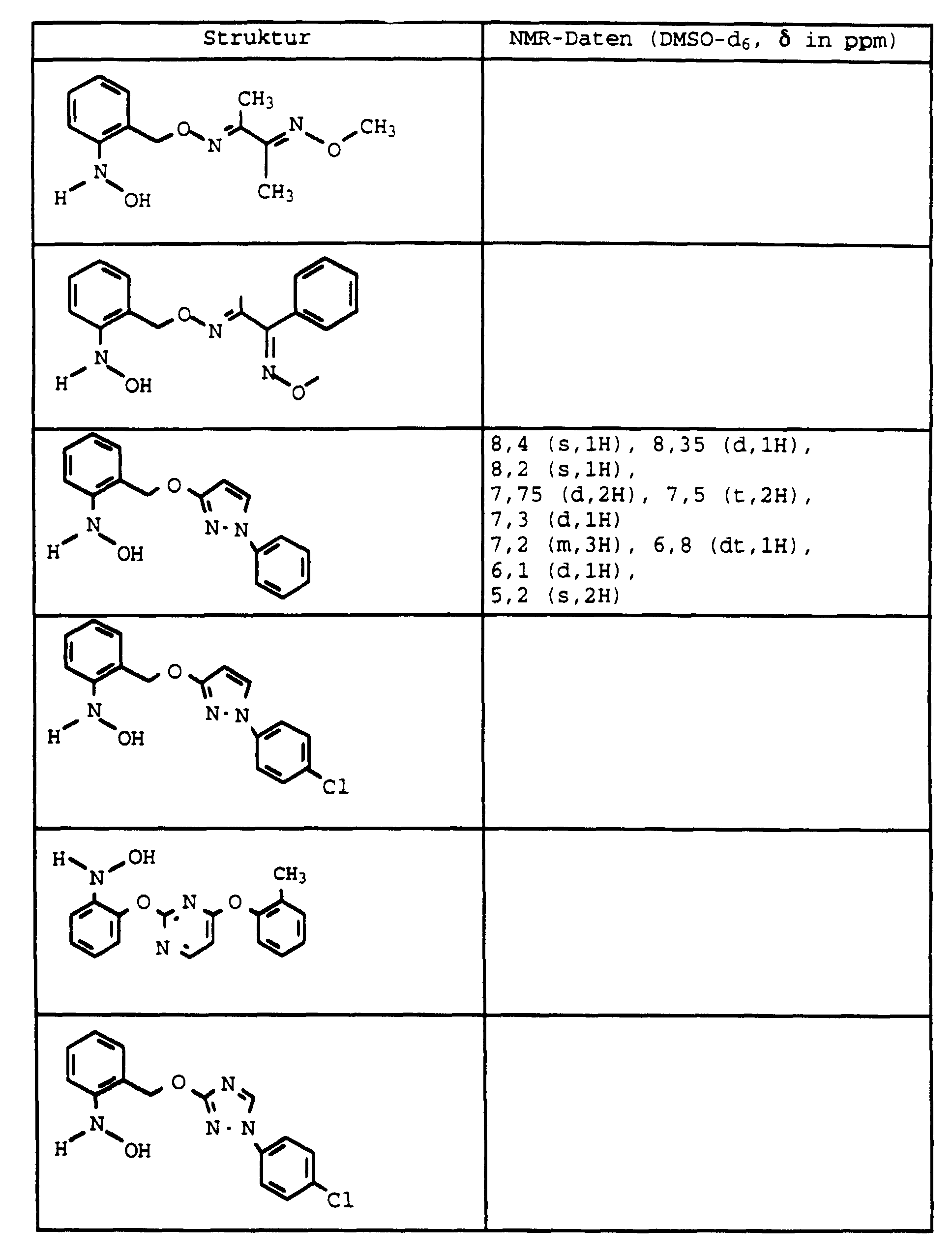

## Patentansprüche

1. Verfahren zur Herstellung von aromatischen oder heteroaromatischen Hydroxylaminen der allgemeinen Formel I 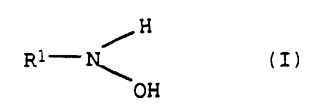 in der R1 einen unsubstituierten oder substituierten Arylrest oder einen unsubstituierten oder substituierten Hetarylrest aus der Gruppe der Pyridine oder Chinoline darstellt, durch Hydrierung von Nitroverbindungen der allgemeinen Formel II
R¹-NO₂ (II)
in der R¹ die oben angegebene Bedeutung hat, in Gegenwart eines Platinkatalysators auf einem Aktivkohleträger oder in Gegenwart eines mit Schwefel oder Selen dotierten Palladiumkatalysators auf einem Aktivkohleträger, dadurch gekennzeichnet, daß die Reaktion in Gegenwart einer am Stickstoffatom substituierten Morpholinverbindung der allgemeinen Formel III durchgeführt wird, 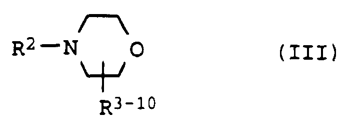 wobei R² Alkylreste mit 1 bis 5 Kohlenstoffatomen und R³ bis R¹⁰ Wasserstoffatome oder Alkylreste mit 1 bis 5 Kohlenstoffatomen bedeuten.

2. Verfahren gemäß Anspruch 1, dadurch gekennzeichnet, daß R¹ für einen substituierten Arylrest oder Hetarylrest steht, der 1 bis 3 Substituenten R¹¹, R¹² und R¹³ tragen kann, wobei R¹¹, R¹² und R¹³ gleich oder verschieden sein können und für folgende Bedeutung stehen:
Wasserstoff, Halogen, C1-C4-Alkyl, Halo-(C₁-C₄)-Alkyl, (C₁-C₄)-Alkoxy, Halo-(C₁-C₄)-Alkoxy, C₁-C₄-Alkylthio, (C₁-C₄)-Alkylcarbonyl, (C₁-C₄)-Alkyl- (CR₁₄=N-O- (C₁-C₄)-Alkyl) (C₁-C₄)-Alkoxycarbonyl, (C₁-C₄)-Alkylaminocarbonyl, (C1-C4)-Dialkylaminocarbonyl, (C1-C4)-Alkylcarbonylamino, (C₁-C₄)-Alkylcarbonyl-(C₁-C₄)-alkylamino, -CH₂O-N=C(R¹⁵)-C(R¹⁶)=N-O-R¹⁷, Cyano oder die Gruppe A - B,
wobei
A -O-, -S-, -O-CH₂-, CH₂-O-, -S-CH₂-, -CH₂-S-, -CH₂-O-CO-, -CH₂-N(R¹⁸)-, -CH=CH-, -CH=N-O-, -CH₂-O-N=C(R¹⁵)- oder eine Einfachbindung und
B Phenyl, Naphthyl, Pyridinyl, Pyrazinyl, Pyrimidinyl, Pyridazinyl, Pyrazolyl, Imidazolyl, Oxazolyl, Isoxazolyl, Thiazolyl, Isothiazolyl, 1,2,4-Triazolyl, 1,2,3-Triazolyl, Furanyl, Thienyl, Pyrrolyl oder C₃-C₇-Cycloalkyl bedeutet, wobei B mit 1 - 3 Substituenten R¹⁹ substituiert sein kann.
R¹⁴ und R¹⁸ bedeuten unabhängig voneinander C₁-C₄-Alkyl, C₂-C₄-Alkenyl, C₂-C₄-Alkinyl oder Wasserstoff,
R¹⁵ und R¹⁷ bedeuten Wasserstoff, C₁-C₄-Alkyl, C₁-C₄-Alkoxy, Cyano, C₁-C₄-Alkylthio, Halogen, Cyclopropyl oder Trifluormethyl und R¹⁹ bedeutet
Wasserstoff
Halogen, C₁-C₄-Alkyl, Halo-(C₁-C₄)-Alkyl, (C₁-C₄)-Alkoxy, Halo-(C₁-C₄)-Alkoxy, C₁-C₄-Alkylthio, (C₁-C₄)-Alkylcarbonyl, (C₁-C₄)-Alkyl- (CR¹⁴=N-O-(C₁-C₄)-Alkyl), (C₁-C₄) -Alkoxycarbonyl, (C₁-C4)-Alkylaminocarbonyl, (C₁-C4)-Dialkylaminocarbonyl, (C₁-C₄)-Alkylcarbonylamino, (C₁-C₄)-Alkylcarbonyl-(C₁-C4)-alkylamino, oder Cyano,
R¹⁶ bedeutet C₁-C₄-Alkyl, C₃-C₆-Cycloalkyl, Phenyl, Hetaryl oder Heterocyclyl.

3. Verfahren gemäß einem der Ansprüche 1 oder 2, dadurch gekennzeichnet, daß als Morpholinverbindung der allgemeinen Formel III 4-Methylmorpholin, 4-Ethylmorpholin, 4-Propylmorpholin, 4-Butylmorpholin, 4-Isobutylmorpholin, 4-Tertiärbutylmorpholin, 4-Pentylmorpholin, 4-Isopentylmorpholin, 2,4,6-Trimethylmorpholin, 2,3,4,5,6-Pentamethylmorpholin oder 2,2,4,6,6-Pentamethylmorpholin verwendet wird.

4. Verfahren gemäß einem der Ansprüche 1 bis 3, dadurch gekennzeichnet, daß das Gewichtsverhältnis von Morpholinverbindung zu Nitroverbindung größer als 1 ist.

5. Verfahren gemäß einem der Ansprüche 1 bis 4, dadurch gekennzeichnet, daß der Platinkatalysator oder der Palladiumkatalysator in einer Menge von 0,1 bis 15 Gew.-%, bezogen auf den Aktivkohleträger verwendet wird.

6. Verfahren gemäß einem der Ansprüche 1 bis 5, dadurch gekennzeichnet, daß der Platinkatalysator oder der Palladiumkatalysator in einer Menge von 0,001 bis 1,0 Gew.-% Platin oder Palladium, bezogen auf die Nitroverbindung verwendet wird.

7. Verfahren gemäß einem der Ansprüche 1 bis 6, dadurch gekennzeichnet, daß die Hydrierung bei einer Temperatur von -20°C bis 100°C durchgeführt wird.

8. Verfahren gemäß einem der Ansprüche 1 bis 7, dadurch gekennzeichnet, daß die Hydrierung bei einem Druck von Normaldruck bis 10 bar Überdruck durchgeführt wird.

## Claims

1. A process for preparing aromatic or heteroaromatic hydroxylamines of the general formula I 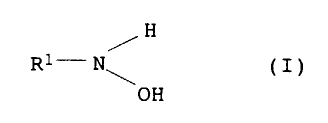 where R¹ is an unsubstituted or substituted aryl radical or an unsubstituted or substituted hetaryl radical from the pyridine or quinoline groups, by hydrogenation of nitro compounds of the general formula II
R¹-NO₂ (II)
where R¹ has the meanings indicated above, in the presence of a platinum catalyst on an activated carbon support or in the presence of a palladium catalyst doped with sulfur or selenium on an activated carbon support, which comprises carrying out the reaction in the presence of a nitrogen-substituted morpholine compound of the general formula III 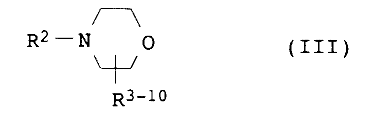 R² being alkyl radicals having 1 to 5 carbon atoms and R³ to R¹⁰ being hydrogen atoms or alkyl radicals having 1 to 5 carbon atoms.

2. A process as claimed in claim 1, wherein R¹ is a substituted aryl radical or hetaryl radical which can carry 1 to 3 substituents R¹¹, R¹² and R¹³, it being possible for R¹¹, R¹² and R¹³ to be identical or different and to have the following meanings:
hydrogen, halogen, C₁-C₄-alkyl, halo-(C₁-C₄)-alkyl, (C₁-C₄)-alkoxy, halo-(C₁-C₄)-alkoxy, C₁-C₄-alkylthio, (C₁-C₄)-alkylcarbonyl, (C₁-C₄)-alkyl-(CR₁₄=N-O-(C₁-C₄)-alkyl), (C₁-C₄)-alkoxycarbonyl, (C₁-C₄)-alkylaminocarbonyl, (C₁-C₄)-dialkylaminocarbonyl, (C₁-C₄)-alkylcarbonylamino, (C₁-C₄)-alkylcarbonyl-(C₁-C₄)-alkylamino, -CH₂O-N=C(R¹⁵)-C(R¹⁶)=N-O-R¹⁷, cyano or the group A - B,
A being -O-, -S-, -O-CH₂-, CH₂-O-, -S-CH₂-, -CH₂-S-, -CH₂-O-CO-, -CH₂-N(R¹⁸)-, -CH=CH-, -CH=N-O-, -CH₂-O-N=C(R¹⁵)- or a single bond and
B being phenyl, naphthyl, pyridinyl, pyrazinyl, pyrimidinyl, pyridazinyl, pyrazolyl, imidazolyl, oxazolyl, isoxazolyl, thiazolyl, isothiazolyl, 1,2,4-triazolyl, 1,2,3-triazolyl, furanyl, thienyl, pyrrolyl or C₃-C₇-cycloalkyl, it being possible for B to be substituted by 1 - 3 substituents R¹⁹,
R¹⁴ and R¹⁸ are independently of one another C₁-C₄-alkyl, C₂-C₄-alkenyl, C₂-C₄-alkynyl or hydrogen,
R¹⁵ and R¹⁷ are hydrogen, C₁-C₄-alkyl, C₁-C₄-alkoxy, cyano, C₁-C₄-alkylthio, halogen, cyclopropyl or trifluoromethyl and R¹⁹ is
hydrogen,
halogen, C₁-C₄-alkyl, halo-(C₁-C₄)-alkyl, (C₁-C₄)-alkoxy, halo-(C₁-C₄)-alkoxy, C₁-C₄-alkylthio, (C₁-C₄)-alkylcarbonyl, (C₁-C₄)-alkyl-(CR¹⁴=N-O-(C₁-C₄)-alkyl), (C₁-C₄)-alkoxycarbonyl, (C₁-C₄)-alkylaminocarbonyl, (C₁-C₄)-dialkylaminocarbonyl, (C₁-C₄)-alkylcarbonylamino, (C₁-C₄)-alkylcarbonyl-(C₁-C₄)-alkylamino, or cyano,
R¹⁶ is C₁-C₄-alkyl, C3-C6-cycloalkyl, phenyl, hetaryl or heterocyclyl.

3. A process as claimed in one of claims 1 and 2, wherein the morpholine compound of the general formula III employed is 4-methylmorpholine, 4-ethylmorpholine, 4-propylmorpholine, 4-butylmorpholine, 4-isobutylmorpholine, 4-tertiarybutylmorpholine, 4-pentylmorpholine, 4-isopentylmorpholine, 2,4,6-trimethylmorpholine, 2,3,4,5,6-pentamethylmorpholine or 2,2,4,6,6-pentamethylmorpholine.

4. A process as claimed in one of claims 1 to 3, wherein the weight ratio of morpholine compound to nitro compound is greater than 1.

5. A process as claimed in one of claims 1 to 4, wherein the platinum catalyst or the palladium catalyst is used in an amount from 0.1 to 15 % by weight, based on the activated carbon support.

6. A process as claimed in one of claims 1 to 5, wherein the platinum catalyst or the palladium catalyst is used in an amount from 0.001 to 1.0 % by weight of platinum or palladium, based on the nitro compound.

7. A process as claimed in one of claims 1 to 6, wherein the hydrogenation is carried out at from -20°C to 100°C.

8. A process as claimed in one of claims 1 to 7, wherein the hydrogenation is carried out at a pressure from normal pressure to 10 bar overpressure.

## Revendications

1. Procédé pour la préparation d'hydroxylamines aromatiques ou hétéroaromatiques de formule générale I 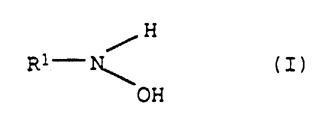 où R¹ représente un reste aryle substitué ou non substitué ou un reste hétaryle substitué ou non substitué du groupe des pyridines ou des quinoléines, par hydrogénation de composés nitrés de formule générale II
R¹-NO₂ (II)
où R¹ a la signification susdite, en présence d'un catalyseur au platine sur un support au charbon actif ou en présence d'un catalyseur au palladium dopé au soufre ou au sélénium sur un support au charbon actif, caractérisé par le fait que la réaction est effectuée en présence d'un composé de morpholine substitué sur l'atome d'azote de formule générale III, 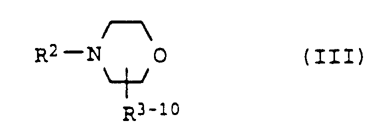 où R² désignent des restes alkyle ayant 1 à 5 atomes de carbone et R³ à R¹⁰ représentent des atomes d'hydrogène ou des restes alkyle ayant 1 à 5 atomes de carbone.

2. Procédé selon la revendication 1, caractérisé par le fait que R¹ désigne un reste aryle ou hétaryle substitué, qui peut porter 1 à 3 substituants R¹¹, R¹² et R¹³, R¹¹, R¹² et R¹³ pouvant être identiques ou différents et ayant la signification suivante :
hydrogène, halogène, alkyle en C₁-C₄, halogéno-alkyle-(C₁-C₄), alcoxy(C₁-C₄), halogéno-alcoxy(C₁-C₄), alkyl-(C₁-C₄)thio, alkyl (C₁-C₄) carbonyle, alkyl (C₁-C₄)-(CR₁₄=N-O-alkyle en C₁-C₄), alcoxy(C₁-C₄)carbonyle, alkyl(C₁-C₄)aminocarbonyle, dialkyl(C₁-C₄)aminocarbonyle, alkyl(C₁-C₄)carbonylamino, alkyl(C₁-C₄)-carbonyl-alkyl(C₁-C₄)amino, -CH₂O-N=C(R¹⁵)-C(R¹⁶)=N-O-R¹⁷, cyano ou le groupe A - B,
dans lequel
A représente -O-, -S-, -O-CH₂-, -CH₂-O-, -S-CH₂-, -CH₂-S-, -CH₂-O-CO-, -CH₂-N(R¹⁸)-, -CH=CH-, -CH=N-O-, -CH₂-O-N=C(R¹⁵)- ou une simple liaison et
B représente un groupe phényle, naphtyle, pyridinyle, pyrazinyle, pyrimidinyle, pyridazinyle, pyrazolyle, imidazolyle, oxazolyle, isoxazolyle, thiazolyle, isothiazolyle, 1,2,4-triazolyle, 1,2,3-triazolyle, furannyle, thiényle, pyrrolyle ou cycloalkyle en C₃-C₇, tandis que B peut être substitué avec 1 - 3 substituants R¹⁹,
R¹⁴ et R¹⁸ désignent indépendamment l'un de l'autre un groupe alkyle en C₁-C₄, alcényle en C₂-C₄, alcynyle en C₂-C₄ ou de l'hydrogène,
R¹⁵ et R¹⁷ désignent l'hydrogène ou un groupe alkyle en C₁-C₄, alcoxy en C₁-C₄, cyano, alkyl(C₁-C₄)thio, halogéno, cyclopropyle ou trifluorométhyle, et
R¹⁹ représente
l'hydrogène ou un groupe halogéno, alkyle en C₁-C₄, halogénoalkyle en C₁-C₄,, alcoxy en C₁-C₄, halogéno-alcoxy en C₁-C₄, alkyl(C₁-C₄)thio, alkyl(C₁-C₄)carbonyle, alkyl-(C₁-C₄)-(CR¹⁴=N-O-alkyle en C₁-C₄), alcoxy(C₁-C₄)-carbonyle, alkyl(C₁-C₄)aminocarbonyle, dialkyl(C₁-C₄)-aminocarbonyle, alkyl (C₁-C₄)carbonylamino, alkyl (C₁-C4)carbonyl-alkyl(C₁-C₄)amino, ou cyano, et
R¹⁶ désigne un groupe alkyle en C₁-C₄, cycloalkyle en C₃-C₆, phényle, hétaryle ou hétérocyclyle.

3. Procédé selon l'une des revendications 1 et 2, caractérisé par le fait qu'on utilise, comme composé de morpholine de formule générale III, la 4-méthylmorpholine, la 4-éthylmorpholine, la 4-propylmorpholine, la 4-butylmorpholine, la 4-isobutylmorpholine, la 4-tert-butylmorpholine, la 4-pentylmorpholine, la 4-isopentylmorpholine, la 2,4,6-triméthylmorpholine, la 2, 3,4,5, 6-pentaméthylmorpholine ou la 2,2,4,6,6-pentaméthylmorpholine.

4. Procédé selon l'une des revendications 1 à 3, caractérisé par le fait que le rapport en poids du composé de morpholine au composé nitré est supérieur à 1.

5. Procédé selon l'une des revendications 1 à 4, caractérisé par le fait que le catalyseur au platine ou le catalyseur au palladium est employé en une quantité de 0,1 à 15% en poids, par rapport au support au charbon actif.

6. Procédé selon l'une des revendications 1 à 5, caractérisé par le fait que le catalyseur au platine ou le catalyseur au palladium est employé en une quantité de 0,001 à 1,0% en poids de platine ou de palladium, par rapport au composé nitré.

7. Procédé selon l'une des revendications 1 à 6, caractérisé par le fait que l'hydrogénation est effectuée à une température de -20°C à 100°C.

8. Procédé selon l'une des revendications 1 à 7, caractérisé par le fait que l'hydrogénation est effectuée à une pression allant de la pression normale à une surpression de 10 bar.
